# DEMANDE DE BREVET EUROPEEN

(11) **EP 1 245 216 A1**
(43) Date de publication de la demande: **02.10.2002**
(21) Numéro de dépôt: 01870061.7
(22) Date de dépôt: 26.03.2001
(51) Int. Cl.: A61H 33/02

(54) **Dispositif de bain relaxant**

(71) Demandeur: Piron, Jean-Pol, 1190 Bruxelles (BE)
(72) Inventeur: Piron, Jean-Pol, 1190 Bruxelles (BE)
(74) Mandataire: Claeys, Pierre

(57) **Abrégé**

Dispositif de bain relaxant dans lequel des buses affleurant à la surface interne de la paroi d'une cuve sont prévues pour émettre au moins un faisceau de lumière colorée, lesdites buses étant agencées pour émettre ledit faisceau de lumière colorée sensiblement au contact d'au moins une partie de l'épiderme d'un utilisateur lorsque ce dernier fait usage du dispositif.

## Description

La présente invention concerne un dispositif de bain relaxant comprenant une cuve et une source lumineuse agencée pour émettre de la lumière ayant au moins deux longueurs d'onde différentes sous forme de faisceau lumineux, ladite cuve étant pourvue de buses affleurant à sa surface interne de façon à être en contact avec au moins une partie de l'épiderme d'un utilisateur, lorsque ce dernier fait usage du dispositif.

Des dispositifs de bain relaxant sont disponibles dans le commerce et consistent généralement en une baignoire munie de buses disposées sur les parois verticales latérales, ces buses étant prévues pour propulser de l'eau vers l'intérieur de la baignoire, ainsi que des buses disposées sur le fond de la baignoire pour propulser de l'air dans la baignoire.

Les buses à eau sont parfois pourvues de fibres optiques qui émettent une lumière à l'intérieur de la baignoire en même temps qu'elles y propulsent de l'eau.

Ce système a comme inconvénient principal que la lumière émise est très diffuse dans le bain et les faisceaux lumineux colorent l'eau, la lumière étant diffractée dans toutes les directions. L'on obtient donc un bain coloré et lumineux; cependant pas d'effet chromothérapeutique notoire n'est observé.

La présente invention a pour objet de réaliser une chromothérapie dans un dispositif de bain relaxant.

L'on sait qu'un faisceau lumineux a une énergie spécifique selon sa couleur et donc sa longueur d'onde.

La présente invention prévoit donc un dispositif de bain relaxant comprenant une cuve et une source lumineuse agencée pour émettre de la lumière ayant au moins deux longueurs d'onde différentes sous forme de faisceau lumineux, ladite cuve étant pourvue de buses affleurant à sa surface interne de façon à être en contact avec au moins une partie de l'épiderme d'un utilisateur, lorsque ce dernier fait usage du dispositif, lesdites buses étant reliées à la source lumineuse sont agencées pour émettre ledit faisceau lumineux vers l'intérieur de la cuve.

Le fait que les faisceaux de lumière soient émis sensiblement au contact d'au moins une partie de l'épiderme de l'utilisateur de ce dispositif de bain relaxant a pour effet surprenant que la lumière colorée appliquée au niveau de l'épiderme produit des effets bénéfiques, qui sont variables en fonction de la couleur de la lumière utilisée, le bleu ayant par exemple un effet apaisant et le rouge un effet stimulant sur les utilisateurs de tels dispositifs. Cette chromothérapie a montré des effets bénéfiques surprenants quand l'application des rayonnements lumineux colorés est effectuée sensiblement au contact de l'épiderme, en particulier le long de la colonne vertébrale de l'utilisateur de façon à obtenir un effet relaxant et produire une sensation de détente et de bien-être.

Le dispositif de bain relaxant de la présente invention permet l'émission de lumière colorée sensiblement au contact d'au moins une partie de l'épiderme de l'utilisateur de façon à obtenir des effets bénéfiques qui sont bien moindres lorsque la lumière colorée est diffuse dans l'eau ou dans l'air. En effet, la lumière émise presque au contact de l'épiderme humide, puisque l'utilisateur est dans une baignoire remplie d'eau, diffuse son énergie de façon très efficace autour du corps de l'utilisateur, un faisceau fin permettant d'obtenir des effets chromothérapeutiques bénéfiques en le disposant dans une buse disposée au fond de la baignoire de façon à être pratiquement au contact de l'épiderme de l'utilisateur.

Le dispositif de bain relaxant de l'invention présente l'avantage de comprendre une source de gaz connectée aux buses de façon à ce que le gaz produit par la source soit propulsé par les buses dans la cuve.

Cette source de gaz est, en particulier, un générateur d'air qui aspire l'air, par exemple, dans l'atmosphère ambiante où est disposé le dispositif de bain relaxant de l'invention et le propulse ensuite dans des canalisations reliées aux buses disposées dans la cuve.

Cela permet de pouvoir effectuer un massage en particulier avec des bulles d'air propulsées par les même buses qui émettent la lumière colorée, or, il a été remarqué que le fait d'émettre de la lumière colorée pratiquement au contact de l'épiderme de l'utilisateur lui procurait une sensation de bien-être et de détente amplifiée, l'énergie lumineuse faible émise par les buses disposées au fond de la baignoire en particulier est efficacement répartie autour du corps de l'utilisateur étendu dans la baignoire et cela provoque une synergie avec le massage produit par les bulles d'air émises par les même buses.

Un autre avantage du dispositif de bain relaxant de l'invention est que la source de gaz ou le générateur d'air comprend un élément chauffant agencé pour chauffer le gaz produit par la source de gaz ou l'air produit par le générateur.

Cela permet en effet de propulser, en particulier de l'air à une température qui soit comprise entre environ 40°C et environ 45°C de façon à maintenir l'eau du bain à une température d'au moins 38°C. Il est en effet déplaisant de prendre un bain qui se refroidirait très vite à cause de la propulsion de bulles d'air froid.

Un autre avantage du dispositif de bain relaxant de l'invention est qu'il comprend une unité de contrôle 7 qui est connectée au générateur d'air et à la source lumineuse ainsi qu'éventuellement à une pompe à eau de façon à pouvoir contrôler les différents paramètres de la propulsion d'air, d'eau et l'émission de lumière. Il est ainsi possible de combiner les différents éléments ou de n'en choisir qu'un, quel qu'il soit, selon les préférences de l'utilisateur du dispositif de bain relaxant de l'invention.

Les buses agencées pour émettre la lumière colorée sont réalisées dans une matière choisie parmi le groupe constitué d'acrylique, de polypropylène, de polyéthylène, de chlorure de polyvinyle.

Ces buses permettent à la lumière colorée d'être émises sous forme d'un faisceau fin et dense pratiquement au contact d'au moins une partie de l'épiderme de l'utilisateur, ce qui améliore l'efficacité de la chromothérapie ainsi réalisée.

La source lumineuse utilisée dans le dispositif de bain relaxant de l'invention est pourvue de moyens de réglage pour faire varier la couleur et l'intensité du faisceau lumineux.

En effet, grâce à ces moyens de réglage, l'utilisateur peut choisir la couleur du faisceau lumineux selon l'effet qu'il veut obtenir, le bleu a un effet reposant, le vert a un effet rééquilibrant, le rouge a un effet stimulant, le jaune a un effet tonifiant, le violet a un effet relaxant et le blanc a un effet revitalisant.

Le dispositif de bain relaxant de l'invention peut aussi comprendre au moins une buse à eau disposée de façon affleurante à la surface interne d'au moins une paroi sensiblement verticale de la cuve, ladite au moins une buse à eau étant agencée pour propulser de l'eau vers l'intérieur de la cuve.

La présence d'éléments permettant la propulsion d'air, d'eau et l'émission de lumière dans la même cuve permet de choisir tous les paramètres désirables pour obtenir une sensation de bien-être et de relaxation complète. La programmation est rendue possible par des moyens pour effectuer une programmation combinée de séquences d'émission de lumière, d'air, d'eau et une programmation séparée de ces séquences.

Il est ainsi possible d'alterner les couleurs, de moduler la propulsion d'eau et/ou d'air, de les interrompre ensemble ou séparément et d'obtenir un massage par l'air et/ou l'eau tout en effectuant la chromothérapie adaptée au choix de l'utilisateur.

D'autres détails et particularités de l'invention ressortiront de la description donnée ci-après, à titre non limitatif, et avec référence aux figures annexées.

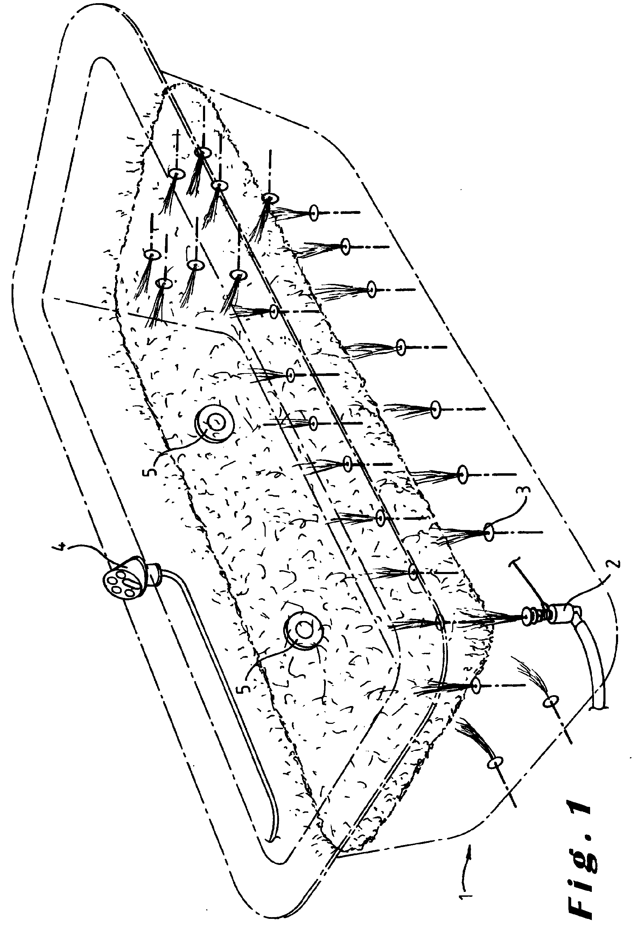

La Figure 1 représente un mode de réalisation préféré d'une partie d'un dispositif de bain relaxant selon la présente invention.

La Figure 2 représente un mode de réalisation préféré des éléments composant un dispositif de bain relaxant suivant l'invention.

La Figure 3 représente une buse du dispositif de bain relaxant, agencée pour émettre de la lumière.

Dans un mode de réalisation préféré d'un dispositif de bain relaxant suivant l'invention, la cuve 1 est une baignoire, celle-ci peut être rectangulaire, ronde ou ovale ou avoir une forme quelconque de façon à pouvoir accueillir au moins un utilisateur. Dans le fond de la baignoire et sur les parois sensiblement verticales antérieure et postérieure de la baignoire, des buses 2 affleurant à la paroi interne de la baignoire sont disposées. Ces buses 2 sont disposées de façon à être au contact de la majeure partie du corps de l'utilisateur, c'est-à-dire ses jambes, son dos, ses épaules, son cou et ses pieds. Les buses 2 sont, par exemple, au nombre de trente, de préférence réparties de la façon suivante : seize buses dans le fond de la baignoire, douze buses sur la paroi sensiblement verticale au niveau du dos de l'utilisateur et deux buses dans la paroi sensiblement verticale au niveau des pieds de l'utilisateur quand celui-ci est allongé dans la baignoire. Cependant, d'autres dispositions des buses 2 sont possibles et un nombre de buses 2 inférieur ou supérieur à trente peut être utilisé, en fonction de la taille et de la conformation de la baignoire dans laquelle ces buses 2 sont disposées.

Les buses 2 disposées sur la paroi verticale au niveau du haut du dos et des épaules de l'utilisateur quand celui-ci est allongé dans la baignoire sont, de préférence disposées par paires symétriques de chaque côté de l'endroit où l'utilisateur appuie sa tête de façon à ce que le faisceau de lumière soit appliqué sur les épaules et au niveau de la nuque. Ces buses 2 agencées pour émettre au moins un faisceau lumineux 3 sont de préférence réalisées en matière acrylique qui allie la solidité à la transparence. Le faisceau lumineux 3 est émis de façon précise au contact de l'épiderme de l'utilisateur et la lumière est diffusée autour du corps dudit utilisateur. Ces buses 2 peuvent diffuser également un jet d'air qui est dirigé dans la baignoire par un mode de diffusion central ou périphérique selon la conformation des buses 2. Ainsi, l'utilisateur peut bénéficier d'un massage par injection d'air dans l'eau du bain qui est combiné ou non avec la diffusion de lumière colorée. Le dispositif de bain relaxant peut comprendre également des buses à eau 5 affleurant à la surface interne des parois latérales de la baignoire. Ces buses à eau 5 sont, de préférence au nombre de quatre sur les parois latérales et au nombre de deux sur la paroi antérieure où l'utilisateur va appuyer ses pieds, mais leur nombre peut varier en fonction de la taille de la baignoire et de la puissance de l'hydromassage que l'on veut obtenir.

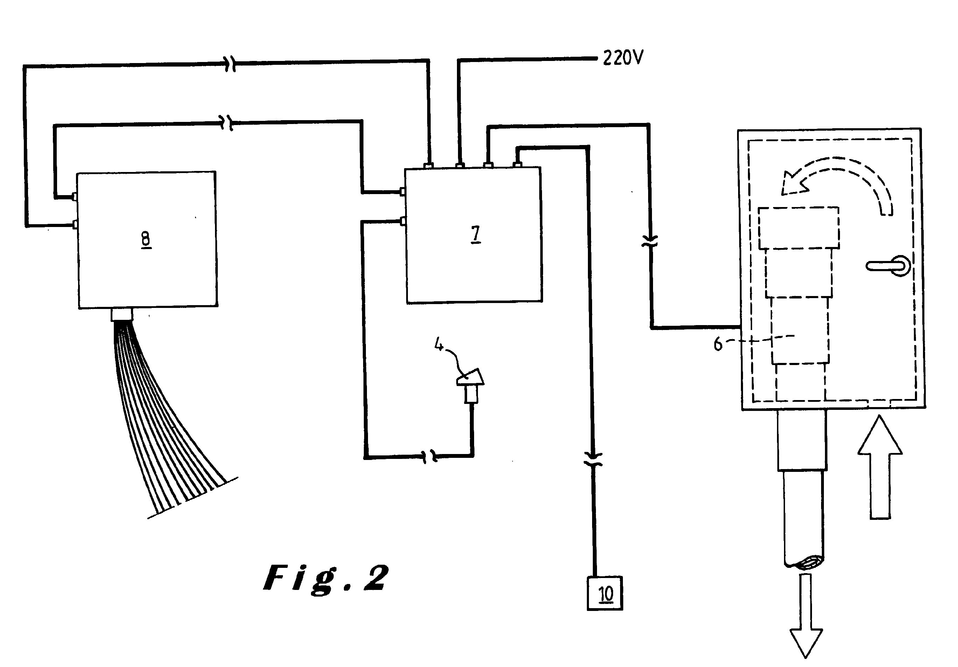

La Figure 2 représente des éléments qui composent le système d'émission de lumière colorée et d'air dans un dispositif de bain relaxant suivant un mode de réalisation préféré, mais ces éléments peuvent être séparés.

Pour aspirer l'air ambiant ou de l'air provenant d'un conteneur, ou même un gaz, un surpresseur d'air 6 dont le débit est compris entre 80 et 140 m³/h est prévu qui est disposé dans une gaine en matière thermoconductrice. Ce surpresseur d'air 6 pourvu d'un filtre microporeux, qui empêche toute pénétration dans les canalisations d'air de particules polluantes (poussières, pollens, spores, etc.), comprend un générateur d'air qui a une puissance choisie entre 200 W et 1200 W, de préférence 900 W et un élément chauffant d'une puissance comprise entre 300 W et 700 W, de préférence de 600 W. L'élément chauffant est muni d'un thermostat pour maintenir la température de l'air aspiré par la turbine entre 40 et 45°C. Cet ensemble générateur d'air ou de gaz constitue une unité technique qui est réalisée de préférence en matériau synthétique structuré, comportant une double isolation acoustique et qui peut être installée sous la baignoire ou à l'écart jusqu'à une distance d'environ 8 m. Cette unité technique est connectée aux buses 2 et elle est commandée par une unité de contrôle 7 permettant de moduler le flux d'air entrant dans la baignoire par les buses 2.

Un générateur de lumière 8 est relié à l'unité de contrôle 7. Ce générateur de lumière 8 comprend une lampe à halogène dont la puissance peut être comprise entre 50 W et 300 W, de préférence 250 W, un transformateur 220/24 V et un disque comprenant toutes les couleurs désirées au faisceau lumineux émis par le générateur de lumière. Le disque est monté sur un moteur qu l'actionne.

La lumière émise par le générateur de lumière est transmise à l'aide de fibres optiques qui se présentent dans un mode de réalisation préféré, en un faisceau de trente brins qui sont chacun positionnés dans une buse 2 de façon à émettre la lumière à l'intérieur de la baignoire par les trente buses 2 prévues dans ce mode de réalisation du dispositif de bain relaxant de l'invention.

Il est bien entendu que le nombre de fibres est corrélé au nombre de buses et qu'une buse peut être munie de deux canaux ou plus afin de disposer au moins deux faisceaux lumineux dans chaque buse.

Un système de massage par eau peut être ajouté au dispositif de bain relaxant comportant un système de chromothérapie et de massage par air. Pour cela, une pompe munie d'équipements de sécurité en conformité avec les normes européennes CEBEC, CE, KEMA, TUV et LCIE est prévue qui permet la circulation de l'eau du bain en circuit fermé et alimente des buses à eau 5 disposées affleurant à la surface interne des parois latérales de la baignoire. Le nombre de buses à eau 5 dépend de la taille et de la forme de la baignoire mais il est, de préférence de quatre buses à eau 5 par paroi latérale auxquelles peuvent être ajoutées des buses à eau 5 sur la paroi contre laquelle l'utilisateur va positionner ses pieds. Une commande électronique 4 est reliée à l'unité de contrôle 7. Cette commande électronique 4, disposée de préférence sur le rebord latéral de la baignoire, permet à l'utilisateur de régler aisément le système de chromothérapie en choisissant la couleur émise ou des séquences de couleurs, ainsi que le débit d'air diffusé par les buses 2 et/ou le débit d'eau propulsé par les buses à eau. Il est également prévu, dans un mode de réalisation de l'invention, de varier la couleur du faisceau lumineux émis dans le bain en suivant le rythme de la musique, cette musicothérapie amplifiant également l'effet relaxant provoqué par la chromothérapie.

Dans le cas ou un hydromassage est prévu dans le dispositif de bain relaxant, un palpeur de niveau 10, qui est un repère électronique de présence ou d'absence d'eau dans la baignoire, est disposé de façon à empêcher la mise en route de la pompe quand la baignoire est vide.

De plus, tous les câbles et toutes les connexions sont complètement isolés pour garantir une sécurité totale à l'utilisateur du dispositif de bain relaxant de l'invention.

Les canalisations sont réalisées, de préférence, dans une matière synthétique inaltérable à faible coefficient de frottement.

Une désinfection des canalisations d'eau est possible après utilisation du dispositif de bain relaxant.

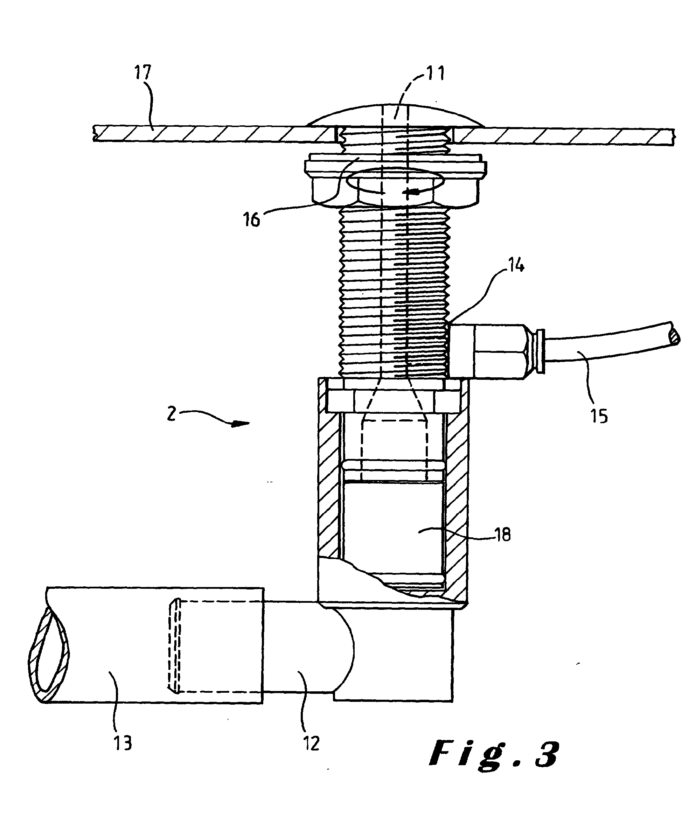

La Figure 3 représente une vue de profil d'une buse 2 par laquelle est émise la lumière colorée ainsi que le jet d'air qui sortent par le même canal 11. La buse 2 comprend une partie basale 12 raccordée à une canalisation d'air 13 et une partie médiane 14 raccordée à au moins une fibre optique 15. La partie terminale 16 de la buse 2 traverse la paroi 17 de la baignoire de façon à affleurer sans constituer une gêne pour l'utilisateur, c'est-à-dire que la surface de la partie terminale 16 de la buse 2 ne présente pas d'aspérités. Une soupape antiretour 18 constitue un mécanisme de sécurité indispensable pour empêcher toute infiltration d'eau dans les canalisations. Ces soupapes antiretour 18 sont présentes dans chaque buse 2.

La fibre optique est connectée sur le côté de la buse, son angle de raccord est quelconque. En effet, étant donné que la buse est réalisée, de préférence dans une matière acrylique, le faisceau lumineux arrivant dans le cylindre interne de la buse est diffracté dans toutes les directions et colore complètement la buse en acrylique, ce faisceau lumineux est ensuite émis au niveau de la partie terminale 16 dans l'eau de la cuve et arrive au niveau de l'épiderme de l'utilisateur allongé dans l'eau.

Ainsi, la lumière émise par toutes les buses 2 enveloppe le corps de l'utilisateur, lui apportant une sensation de bien-être, de relaxation ou de stimulation selon la couleur de la lumière émise. Le fait que la lumière soit émise par les buses 2 qui sont également prévues pour émettre de l'air est important car ces buses 2 sont toujours disposées dans le fond de la baignoire, les bulles d'air s'élevant pour aller au contact du corps de l'utilisateur. Grâce à cette configuration, la lumière émise par ces buses 2 vient également pratiquement au contact de l'épiderme de l'utilisateur, principalement le long de sa colonne vertébrale qui présente des points d'acupuncture qui sont favorables à la réception de signaux, que ce soient des signaux tactiles ou lumineux. De cette façon, la lumière émise plonge l'utilisateur dans un bain de chromothérapie favorable à son bien-être, qui peut encore être augmenté en y ajoutant un massage par air et par eau de façon simple et efficace.

La baignoire utilisée pour ce dispositif de bain relaxant peut être d'une couleur claire ou foncée selon les effets chromothérapeutiques que l'on veut favoriser. En effet les faisceaux colorés émis dans une baignoire de couleur foncée ne présentent pas la même intensité que ceux émis dans une baignoire blanche.

Le dispositif de bain relaxant préférentiel de l'invention prévoit une baignoire, mais ce dispositif d'émission de lumière colorée peut également être disposé sans sortir du cadre de l'invention dans des spas, des pédiluves, des cabines de hammam, des cabines de sauna, des solariums afin de bénéficier de la chromothérapie dans différents environnements également favorables à la détente.

D'autres détails et particularités de la présente invention sont décrits dans les revendications annexées.

## Revendications

1. Dispositif de bain relaxant comprenant une cuve (1) et une source lumineuse agencée pour émettre de la lumière ayant au moins deux longueurs d'onde différentes sous forme de faisceau lumineux (3), ladite cuve étant pourvue de buses (2) affleurant à sa surface interne de façon à être en contact avec au moins une partie de l'épiderme d'un utilisateur, lorsque ce dernier fait usage du dispositif, **caractérisé en ce que** lesdites buses (2) sont reliées à la source lumineuse et sont agencées pour émettre ledit faisceau lumineux (3) vers l'intérieur de la cuve.

2. Dispositif de bain relaxant suivant la revendication 1, **caractérisé en ce qu'**il comprend une source de gaz connectée aux buses (2) de façon à ce que le gaz produit par la source soit propulsé par lesdites buses dans la cuve.

3. Dispositif de bain relaxant suivant la revendication 2, **caractérisé en ce que** la source de gaz est agencée pour produire de l'air.

4. Dispositif de bain relaxant suivant la revendication 2 ou 3, **caractérisé en ce que** la source de gaz, en particulier le générateur d'air, comprend un élément chauffant agencé pour chauffer l'air produit par la source de gaz, en particulier le générateur d'air.

5. Dispositif de bain relaxant suivant l'une des revendications 1 à 4, **caractérisé en ce que** la source de gaz ou le générateur d'air et la source lumineuse sont connectés à une unité de contrôle (7).

6. Dispositif de bain relaxant suivant l'une quelconque des revendications 1 à 5, **caractérisé en ce qu'**une connexion entre la source lumineuse et les buses (2) est constituée de fibres optiques.

7. Dispositif de bain relaxant suivant la revendication 1 ou 2, **caractérisé en ce que** les buses (2) sont constituées d'une matière choisie parmi le groupe constitué d'acrylique, de polypropylène, de polyéthylène, de chlorure de polyvinyle.

8. Dispositif de bain relaxant suivant l'une quelconque des revendications 1 à 7, **caractérisé en ce que** la source lumineuse est pourvue de moyens de réglage pour faire varier la couleur et l'intensité du faisceau lumineux (3).

9. Dispositif de bain relaxant suivant l'une quelconque des revendications 1 à 8, **caractérisé en ce qu'**il comprend également au moins une buse à eau (5) disposée de façon affleurante à la surface interne d'au moins une paroi sensiblement verticale de la cuve, ladite au moins une buse à eau (5) étant agencée pour propulser de l'eau vers l'intérieur de la cuve.

10. Dispositif de bain relaxant suivant l'une quelconque des revendications 1 à 9, **caractérisé en ce qu'**il est pourvu de moyens pour une programmation combinée de séquences d'émission de lumière, d'air, d'eau et une programmation séparée desdites séquences.
